## Europäisches Patentamt

(19) **European Patent Office**

**Office européen des brevets**

(11) Publication number: **0 176 320 B1**

(12) **EUROPEAN PATENT SPECIFICATION**

(45) Date of publication of patent specification: **25.07.90**

(21) Application number: **85306665.2**

(22) Date of filing: **19.09.85**

(84) A request pursuant to Rule 88 EPC for correction of the claims was filed on 31.10.1985.

(51) Int. Cl.⁵: **C 12 N 15/00,** C 12 P 21/02, C 12 N 1/20 // C12R1:07, C12R1:125, C12R1:445, C12R1:19

(54) A method for expression and secretion in bacillus.

(30) Priority: **26.09.84 US 654435**

(43) Date of publication of application: **02.04.86 Bulletin 86/14**

(45) Publication of the grant of the patent: **25.07.90 Bulletin 90/30**

(84) Designated Contracting States: **BE CH DE FR GB IT LI NL SE**

(56) References cited: **EP-A-0 077 664**

**GENE, vol. 22, 1983, Amsterdam; D. SHORTLE "A genetic system for analysis of staphylococcal nuclease"**

**JOURNAL OF BACTERIOLOGY, vol. 157, no. 3, March 1984, Washington D.C.; C.W. SAUNDERS et al. "Use of Chromosomal Integration in the Establishment and Expression of blaZ, a Staphylococcus aureus B-Lactamase Gene, in Bacillus subtilis", pages 718-726**

**Decision of the Technical Board of Appeal 3.3.2, no. T 269/87 of 24 January 1989**

(73) Proprietor: **ELI LILLY AND COMPANY Lilly Corporate Center Indianapolis Indiana 46285 (US)**

(72) Inventor: **Kovacevic, Steven 5620 Broadway Indianapolis Indiana 46220 (US)** Inventor: **Miller, James Robert 9244 Thrushwood Lane Indianapolis Indiana 46250 (US)**

(74) Representative: **Hudson, Christopher Mark et al Erl Wood Manor Windlesham Surrey GU20 6PH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# EP 0 176 320 B1

**Description**

The present invention comprises a novel method for expressing and secreting a functional polypeptide in *Bacillus* and includes vectors and transformants for the practice thereof. These vectors comprise the transcriptional and translational activating sequence and, optionally, the signal peptide coding sequence of the *Staphylococcus aureus* nuclease gene and also a DNA sequence that codes for a functional polypeptide. The vector components are ligated such that the polypeptide is expressed and, optionally, secreted upon appropriate transformation.

The present invention provides a method and associated vectors for the expression and secretion of useful polypeptides in *Bacillus* and other host cells. Prior to the present invention, the development and exploitation of recombinant DNA technology in *Bacillus* has been slow and made especially difficult because of the general lack of suitable expression methods and vectors. This lack of expression methods and vectors is explained in part because foreign transcription and translation initiation signals were not well recognized by *Bacillus*. Consequently, the well known *trp* (Hallewell. R.A . and S. Emtage, 1980, *Gene* 9:27), *lac* (Guarante, L. *et al.,* 1980, Cell 20:543 and Roberts, T. M. *et al.,* 1979, *Proc. Nat. Acad. Sci, USA* 76:5596), *lpp* (Lee, N. *et al.,* 1981 *J. of Bacteriol.* 146:861; Zwiebel, L. J. *et al.,* 1981 *J. of Bacteriol.* 145:654 and Natamura, K. and M. Inouye, 1979, *Cell,* 18:1109) and Bacteriophage λP$_L$ (Derom, C. *et al.,* 1982, *Gene* 17:45; Remaut, E. *et al.,* 1981, *Gene* 15(1):81 and Bernard, H. *et al.,* 1979, *Gene* 5:59) transcription and translation-directing promoter systems are not functional in *Bacillus.* Thus, with the exception of a few drug resistance genes, few foreign and practically no eukaryotic genes have been expressed in *Bacillus.*

The extremely limited ability of *Bacillus* to recognize transcription and translation signals presently available necessitates the development of new sequences that direct gene expression. Several early attempts at expression include the cloning and expression in *B. subtilis* of the *B. licheniformis* betalactamase gene (disclosed in European Patent Office Publication No. 0036259) and the *B. stearo-thermophilus* and *B. amyloliquefaciens* α-amylase genes (disclosed, respectively, in European Patent Office Publication No. 0057976 and Derwent Abstract [Belgium Patent Application No., BE 891—659] No. 37323 E/19). Modifications of the *B. subtilis veg* promoter and translation signals (disclosed in United States Patent Application Serial No. 458,792 (equivalent to GB Publ. No. 2133797)) also have been shown useful for directing the expression of heterologous polypeptides in *Bacillus.* In addition, Palva *et al.,* (Palva *et al.,* 1983, *Gene* 22:229 and Palva *et al.,* 1982, *Proc. Natl. Acad. Sci. USA* 79:5582) have succeeded in expressing and secreting foreign gene products in *B. Subtilis* by using transcription, translation and secretion signals from the B. *amyloliquefaciens* α-amylase gene. About 20 mg of *E. coli* β-lactomase and 500 µg of human interferon per liter were obtained from the culture supernatants. Mosback (Mosback *et al.,* 1983, *Nature* 302:543) has cloned and obtained expression of rat "proinsulin-like" activity at a low level of about 10 µg per liter. Saunders (Saunders *et al.,* 1984, *J. Bacteriol.* 157:718) reported that a *Staphylococcus auerus* β-lactamase was expressed as one percent of the total protein in *B. subtilis.* The β-lactamase protein, normally secreted in *S. aureus,* was not secreted but was cell-associated in *B. subtilis.* Also, Fairweather, (Fairweather *et al.,* 1983, *Infec. Immun.* 41:1112) detected *S. aureus* α-hemolysin in *B. subtilis* supernatants, but this cloned gene has not been sequenced and little is known about the α-hemolysin protein.

Staphylococcus nuclease, one of the most extensively studied enzymes physically and biochemically, is produced and secreted by *Staphlococcus aureus*. This enzyme recently has been cloned and expressed in *E. coli* (Shortle, *Gene* 22:181), but the expression was disappointingly low and the protein was apparently not processed efficiently. As presently disclosed, the *Staphylococcal* nuclease gene, including the signal peptide and nuclease A and B coding regions, has been cloned into *Bacillus subtilis* and shown to be expressed, secreted and properly processed. Biologically active nuclease has been expressed at relatively high levels and analysis by Western blotting demonstrated that the nuclease is secreted into the culture medium and processed to a lower molecular weight protein (nuclease A) while intracellular (or cell-bound) material was not processed and was present as a higher molecular weight nuclease. The Staphylococcal nuclease gene transcriptional, translational and secretional signals are fully functional in *Bacillus* and therefore can be used for the expression and secretion of *Staphylococcal* nuclease or any other commercially important polypeptide. This represents a significant advance in the technical art and helps fill the acute need for expression and secretion methods and vectors for use in *Bacillus* and other gram positive microorganisms.

Gene cloning and expression of products in *Bacillus subtilis* are highly advantageous since the organism is non-pathogenic, does not produce endotoxins and can secrete gene products into the growth medium. In addition, *B. subtilis* has been studied extensively and is the archetype for genetic studies among gram positive microorganisms. The method and expression vectors of the present invention are particularly important because they allow for the commercial exploitation of these important advantages.

For purposes of the present invention, the following terms are defined:

Recombinant DNA Expression Vector — any replicating or integrating agent, including but not limited to plasmids, comprising a DNA molecule to which one or more additional DNA segments can or have been added.

Transformation — the introduction of DNA into a recipient host cell.

Transformant — a recipient host cell that has undergone transformation.

2

Restriction Fragment — any linear DNA generated by the action of one or more restriction enzymes.

Transcriptional Activating Sequence — a DNA sequence that directs the transcription of DNA into messenger RNA (m-RNA).

Translational Activating Sequence — a DNA sequence, including the nucleotide triplet that codes for the translational start codon, that directs the translation of m-RNA into a polypeptide.

Functional Polypeptide — a recoverable bioactive entirely heterologous or homologous polypeptide or precursor, a recoverable bioactive polypeptide comprising a heterologous polypeptide and a portion or whole of a homologous polypeptide, or a recoverable bioinactive fusion polypeptide comprising a heterologous polypeptide and a bioinactivating homologous polypeptide which can be specifically cleaved.

Fused Gene Product — a recoverable heterologous polypeptide which is fused with a portion or whole of a homologous or a different heterologous polypeptide.

The following figures will help to illustrate the invention as further disclosed below:

Figure 1 — Restriction Site Map of Plasmid pOW440

Figure 2 — Restriction Site Map of Plasmid pOW448

Figure 3 — Restriction Site Map of Plasmid pOW341

In particular, the present invention provides a method for expressing a functional polypeptide in *Bacillus*, which comprises:

a) transforming a *Bacillus* host cell with a recombinant DNA expression vector which is selectable and capable of replication in said host cell, said vector comprising

1) the transcriptional and translational activating sequence of the *Staphylococcus aureus* nuclease gene and

2) a DNA sequence that codes for a functional polypeptide; and

b) culturing the transformed cell under conditions suitable for expression of said polypeptide;

subject to the limitation 1) that the functional polypeptide sequence and the transcriptional and translational activating sequence are immediately adjacent, in translational reading frame and positioned for expression of said functional polypeptide and 2) that the functional polypeptide sequence is exclusive of the nucleotide triplet that codes for the N-terminal amino acid of said functional polypeptide when said amino acid is methionine. The invention further provides the previously described method in which

a) the recombinant DNA expression vector further comprises a signal peptide coding sequence, and

b) in which said transformed cells are cultured under conditions suitable for expression and secretion of said functional polypeptide,

subject to the limitation 1) that the signal peptide coding sequence is exclusive of the nucleotide triplet that codes for the N-terminal amino acid of said signal peptide when said amino acid is methionine and that said signal peptide coding sequence is positioned for expression immediately adjacent, downstream and in the translational reading frame of said transcriptional and translational activating sequence, 2) that the nucleotide triplet coding for the C-terminus of the signal peptide encoded by said signal peptide coding sequence is immediately adjacent to, upstream of and in translational reading frame with the functional polypeptide sequence, and 3) that the functional polypeptide sequence is inclusive of the nucleotide triplet that codes for the N-terminal amino acid of said functional polypeptide. The present invention also provides the related recombinant DNA expression vectors and transformants.

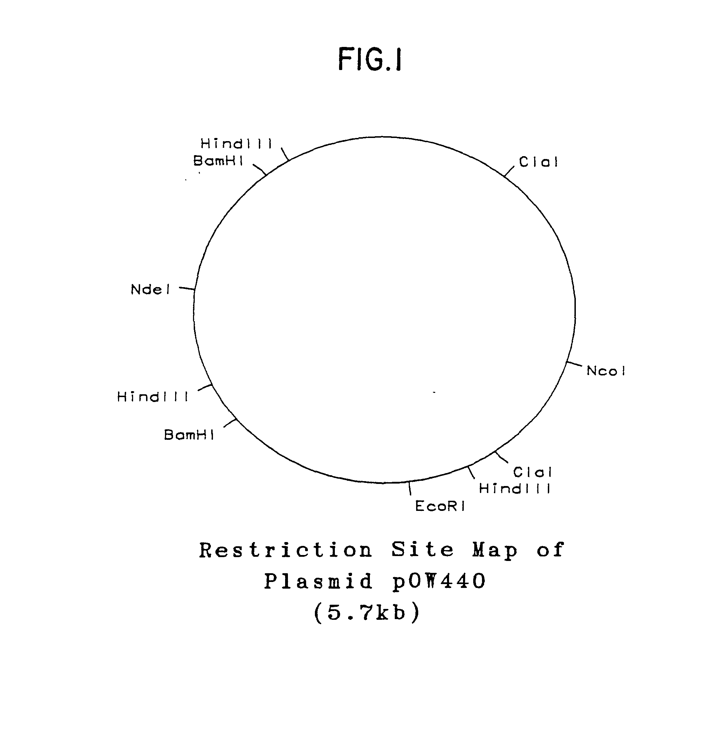

The method of the present invention is exemplified by constructing recombinant DNA expression vectors that code for the expression and secretion of either Staphylococcal nuclease or other functional polypeptides in *Bacillus*. The aforementioned nuclease gene was cloned in such a way as to convert the plasmid pFOG301 (Shortle, 1983) Staphylococcal nuclease gene-containing ~1.4 kb *Hpa*II fragment into a *Bam*HI fragment. The actual cloning was performed by filling in both the *Bam*HI site of plasmid pBR322 and the isolated *Hpa*II fragment of plasmid pFOG301 by use of Klenow enzyme and then ligating the resultant flush-ended fragments. All Ap$^r$ clones were screened for the production of nuclease by a chromogenic plate test. Approximately 15 percent gave a positive indication of nuclease activity. Plasmids from the nuclease positive clones were shown to contain a distinctive *Bam*HI fragment of the same size as the original *Hpa*II fragment. One such plasmid, designated as plasmid pOW50, was *Bam*HI digested and the resultant fragments ligated to similarly digested plasmid pOW430. The resultant plasmid, designated as plasmid pOW440, contains the Staphylococcal nuclease transcriptional, translational, secretional and structural coding sequences. Plasmid pOW440 is functional and codes for the expression and secretion of Staphylococcal nuclease in *Bacillus* and thus can be used conveniently to exemplify the present method. A restriction site map of plasmid pOW440 is presented in Figure 1 of the accompanying drawings.

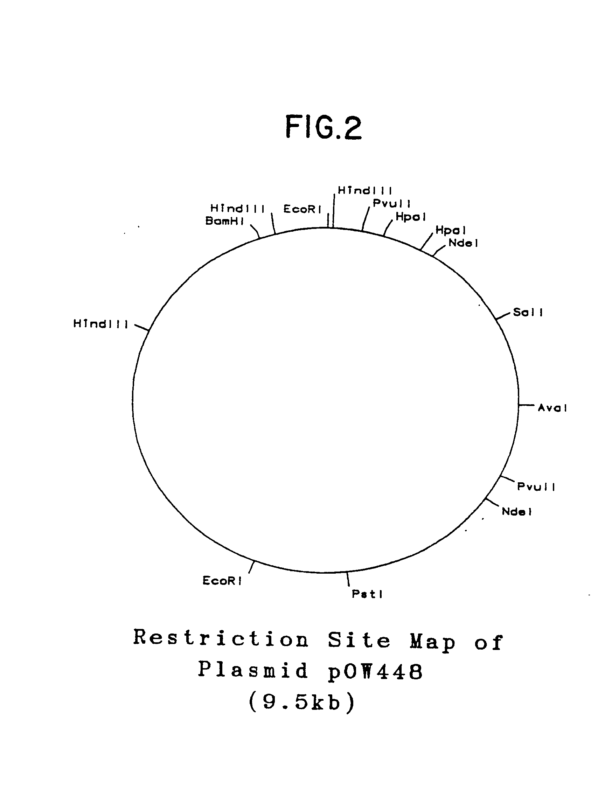

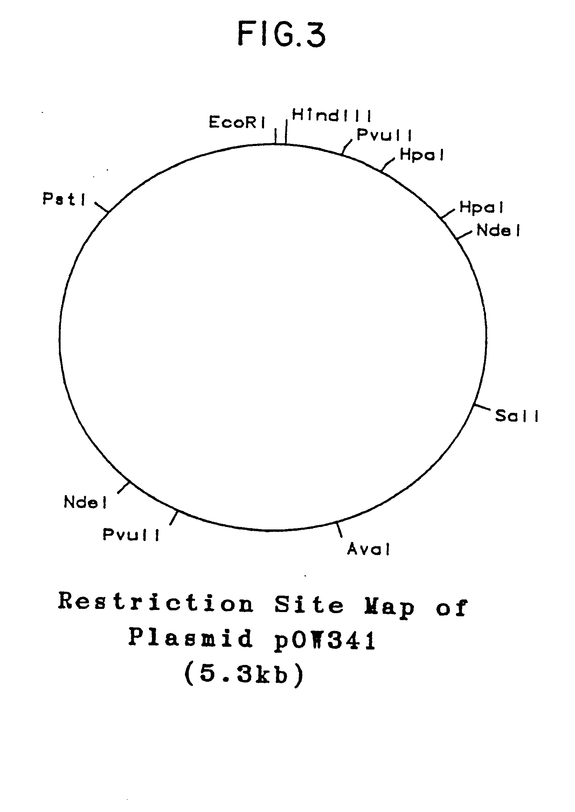

The present invention is further exemplified by constructing recombinant DNA expression vectors that code for the expression and secretion of human proinsulin. This was done by ligating the ~.295 kb *Bam*HI fragment of plasmid pOW340 into *Bgl*II-digested plasmid pOW650. The resulting plasmid, designated as pOW341, was then *Eco*RI digested and ligated to similarly digested plasmid pOW430 to form the desired plasmid pOW448. Plasmid pOW448 contains the structural gene for human proinsulin covalently linked in translational reading frame with the signal peptide coding and the transcriptional and translational activating sequences of the *Staphylococcus aureus* nuclease gene. Plasmid pOW448 is functional and codes for the expression and secretion of a human proinsulin product in both *E. coli* and *Bacillus* and thus can conveniently be used to exemplify the present method. A restriction site map of plasmids pOW448 and

EP 0 176 320 B1

pOW341, respectively, is presented in Figures 2 and 3 of the accompanying drawings.

The starting materials and certain vectors used to exemplify the present invention are readily available or can be constructed following known procedures. Plasmid pOW440, for example, can be obtained from *Bacillus subtilis* MI112/pOW440, a strain deposited and made part of the permanent stock culture collection of the Northern Regional Research Laboratory, Peoria, Illinois. The strain is available as a preferred source and stock reservoir of the plasmid under the accession number NRRL B—15887. The starting material plasmid pOW340 is constructed by ligating 1) the DNA linker sequence

```
5' GAT CCA ACA GTA TAT AGT GCA ACT TTC GTT AAC CAA CAC TTG T 3'
        || ||| ||| ||| ||| ||| ||| ||| ||| ||| ||| ||| |||
3'     GT TGT CAT ATA TCA CGT TGA AAG CAA TTG GTT GTG AAC  5'
```

wherein

A is deoxyadenyl,

G is deoxyguanyl,

C is deoxycytosyl and

T is thymidyl;

2) the ~.27 kb *Hph*I-*Xho*II fragment of plasmid pNM587.4—4, and 3) *Bam*HI-digested plasmid pUC8. Plasmid pNM587.4—4 can be obtained from *E. coli* K12 JA221/pNM587.4—4, a strain deposited and made part of the stock culture collection of the Northern Regional Research Laboratory, Peoria, Illinois. It is available as a preferred source and stock reservoir of the plasmid under the accession number NRRL B—15812. Plasmid pUC8 is commercially available from Bethesda Research Laboratories, P.O. Box 6009, Gaithersburg, Maryland. The above linker sequence can be synthesized conventionally by use of known apparatus, such as, for example, the DNA Synthesizer 380A of Applied Biosystems, Foster City, California or can be synthesized in accordance with the procedures of Itakura *et al.*, 1977, *Science* 198:1056 and Crea *et al.*, 1978, *Proc. Nat. Acad. Sci.* USA 75:5765.

The plasmid pOW650 starting material is constructed by ligating the ~.65 kb *Xho*II fragment of plasmid pOW440 into *Bam*HI/*Bgl*II-digested plasmid pKC7. Plasmid pKC7 can be obtained from the American Type Culture Collection, Rockville, Maryland and is available without restriction under the accession number ATCC 37084. The plasmid pOW430 starting material is a cloning vector that contains a *Bacillus*-functional origin of replication and also appropriate selectable markers for use in *Bacillus*. Plasmid pOW430 can be obtained from *Bacillus subtilis* MI112/pOW430, a strain deposited at the aforementioned Northern Regional Research Laboratory under the accession number NRRL B—15833.

The illustrative plasmid pOW448 codes for the expression and secretion in *Bacillus* of human proinsulin product. Secretion occurs because the vector coding for proinsulin also codes for the *Staphylococcus aureus* nuclease signal peptide. Signal peptides are short leader regions of amino acids. which often comprise newly synthesized polypeptides and which are believed to function in the transport of polypeptides across cell membranes. Signal peptides typically are cleaved from the newly synthesized polypeptides during transport, liberating the desired functional polypeptide in the culture medium. Those skilled in the art will recognized that the present invention is not limited to the use of the aforementioned nuclease signal peptide coding sequence but that various secretory signal peptide sequences can be substituted. Such secretion coding sequences include, but are not limited to, the α-amylase signal peptide sequence of *B. amyloliquifaciens* (disclosed in Palva *et al.*, 1981, *Gene* 15:43 and Palva *et al.*, 1982, *Proc. Nat. Acad. Sci. USA* 79:5582), the β-lactamase Type I signal peptide sequence of *B. cereus* (disclosed in Sloma and Gross, 1983, *Nucleic Acids Res.* 11:4997 and Mezes *et al.*, 1983, *FEBS Lett.* 161:195, the *B. subtilis* levansucrase signal peptide sequence (dislcosed in Forret *et al.*, 1984, *Biochem. Biophys. Res. Comm.* 119:795) and the *B. amyloliquefaciens* subtilisin signal peptide sequence (disclosed in Wells *et al.*, 1983, *Nucleic Acids Res.* 11:7911). The above secretory coding sequences can be appropriately ligated to the transcriptional and translational activating sequence of the *S. aureus* nuclease gene and also to a sequence that codes for a functional polypeptide. The resultant expression and secretion sequence can be used as a 'cassette' for constructing vectors that further exemplify the present invention.

Skilled artisans will recognize that the transcriptional and translational activating sequence of the *Staphylococcus* nuclease gene can be ligated also directly to a sequence coding for a fucntional polypeptide. Such constructions lack a signal peptide coding sequence and thus, upon appropriate transformation, result in intracellular expression of product. Under such conditions the functional polypeptide accumulates within the host cell and may not be secreted into the culture medium. Products produced in this way can be isolated by conventional extraction and purification techniques (Methods of Enzymology XXII and XXXIV, Academic Press, New York, New York and EPO Publication Number 0111814, section 5.6) widely used in the fermentation industry. In addition, other purification techniques such as standard chromatography, including cation or anion exchange, sizing resins or bound antibody [affinity], or centrifugation can be used.

The previously described vectors that express but do not secrete functional polypeptides are best constructed by synthetically reconstructing the present transcriptional and translational activating sequence so that the ATG translational start signal is contained within the recognition sequence for a restriction enzyme at the 3' end. The enzymes *Nco*I, *Nde*I, *Sph*I and *Nsi*I (or their isoschizomers) recognize

4

such sequences and are commercially available. Such a synthetically constructed fragment then is inserted into a plasmid vector which contains a unique site for one of these enzymes (e.g. pBR328 and pOW430 both have a unique *Ncol* site and replicate in *E. coli* and *B. subtilis,* respectively) with the upstream (5′) end being inserted at another restriction enzyme recognition site (e.g. *Eco*RI or BamHI). Ligation and transformation with the recombinant plasmid will regenerate the unique *Ncol* restriction site. The coding sequence for a functional polypeptide then may be built by a combination of synthetic linkers and purified fragments such that upon ligation and transformation into the bacterial cell, the coding sequence for the functional polypeptide is in the proper orientation and reading frame for expression. More particularly, a sequence coding for human proinsulin may be inserted into a vector by ligating a linker with *Ncol* and *Hphl* ends, for example,

$$5' \quad \text{CATGTTC GTT AAC CAA CAC TTG T} \quad 3'$$
$$\text{||| ||| ||| ||| ||| |||}$$
$$3' \quad \text{AAG CAA TTG GTT GTG AAC} \quad 5',$$

with the unique ~.27 kb *Hphl—Xholl* fragment of plasmid pNM587.4—4 and then combining with an appropriate vector restricted such that it recognizes *Ncol* and *Bam*HI staggered ends. Those skilled in the art can select any number of plasmid vectors available with the proper sites. Alternatively, one may select a convenient, available restriction site within the signal peptide coding sequence that interrupts the secretion function. Insertion of a proper linker will allow for the ligation of coding sequences for the expression of fused gene products. A *Ndel* site, located in the Staphylococcal signal sequence about 30 base pairs from the initiation sequence, can be used conveniently for such constructions.

The present invention is particularly versatile and can be applied to the production of any functional polypeptide encoded in a recombinant DNA expression vector. A preferred recombinant DNA expression vector is the plasmid although bacteriophage and other useful vectors will be apparent to those skilled in the art. In addition, various sequences that code for functional polypeptides can be substituted for the illustrative Staphylococcal nuclease and human proinsulin coding sequences specifically exemplified. Such sequences include those that are naturally occurring, non-naturally occurring and those that are in part naturally occurring and in part synthetic or non-naturally occurring. More particularly, illustrative sequences can code for human insulin A-chain, human insulin B-chain, non-human insulin A-chain, non-human insulin B-chain, human growth hormone, non-human growth hormone, bovine growth hormone, porcine growth hormone, human interferon, non-human interferon, viral antigen, urokinase, human tissue plasminogen activator, interleukin I, interleukin II, growth hormone releasing factor, any hormone, any enzyme or virtually any other polypeptide with research or commercial value.

The recominant DNA expression vectors and method of the present invention are not limited for use in a single species or strain. To the contrary, the vectors and method are broadly applicable and can be employed using host cells of many taxa, particularly the restrictionless strains of *Bacillus, Staphylococcus* and *E. coli.* Restrictionless strains are selected and isolated readily from *Bacillus* and other taxa by conventional procedures and extensions of principles well known in the art (Lomovskaya *et al.,* 1980, *Microbiological Reviews* 44:206). Host cells of restrictionless strains lack restriction enzymes and, therefore, do not cut or degrade plasmid DNA upon transformation. For purposes of the present application, host cells containing restriction enzymes that do not cut any of the restriction sites of the present vectors also are considered restrictionless.

Preferred host cells of restrictionless strains of *Bacillus,* in which the present method and vectors are especially useful, include restrictionless cells of, for example, *B. subtilis, B. subtilis* MI112, *B. subtilis* SR22, *B. thuringiensis, B. thuringiensis* var. *israeliensis, B. cereus, B. anthracis, B. piliformis, B. tropicus, B. alvei, B. megaterium, B. pumilus, B. licheniformis, B. polymyxa, B. macerans, B. circulans, B. stearothermophilus, B. coagulans, B. firmus, B. brevis, B. sphaericus, B. pasteurii, B. fastidiosus, B. larvae, B. lentimorbus, B. apiarus, B. amyloliquifaciens, B. laterosporus,* and *B. popillae.*

Preferred host cells of restrictionless strains of *Staphylococcus* taxa in which the present method and vectors are useful include restrictionless cells of, for example, *S. aureus, S. carnosus, S. epidermidis,* and *S. saprophyticus.* The invention is not limited for use in *Bacillus* and *Staphylococcus* but can also be used in various *E. coli* host cells. Preferred *E. coli* host cells include, but are not limited to, *E. coli* K12, *E. coli* K12 JA221, *E. coli* K12 HB101, *E. coli* K12 C600, *E. coli* K12 C600$M_k R_k^-$, *E. coli* K12 C600$M_k^- R_k^-$, *E. coli* K12 C600$M_k^+ R_k^-$ and *E. coli* K12 RV308.

While all the embodiments of the present invention are useful, some of the present expression vectors are preferred. Accordingly, preferred vectors are plasmids pOW440 and pOW448 and preferred transformants are *Bacillus subtilis* MI112/p440, *B. subtilis* MI112/pOW448, *B. subtilis* SR22/pOW448 and *B. subtilis* SR22/pOW440. Of this preferred group, plasmid pOW448 and transformant *B. subtilis* SR22/pOW448 are most preferred.

The recombinant DNA expression vectors and transformants of the present invention have broad utility and help fill the need for expression vehicles, especially for use in *Bacillus.* The present invention thus allows for the genetic expression and secretion in *Bacillus* of an assortment of important products including those now bioproduced in *E. coli.* This is especially advantageous because large scale fermantation of *Bacillus* is better known and understood than is fermentation of *E. coli*. In fact, commercial fermentation of *E. coli* is still highly experimental and fraught with difficulty. The present invention circumvents this problem by providing the alternative of producing compounds (some of which are now biosynthesized in *E. coli*) such as, for example, human insulin A-chain, human insulin B-chain, human proinsulin, growth hormone and the like in *Bacillus*. This is true because the present vectors are highly versatile and can accommodate DNA sequences that encode virtually any functional polypeptide. Thus, the present invention allows for flexibility in the choice of hosts and provides a means for using *Bacillus* in the bioproduction and secretion of polypeptides and other gene products.

The ability of the present transformants to secrete polypeptide products is commercially advantageous. For example, isolation and purification of polypeptides can be done continuously during fermentation without the lytic destruction of host cells. Secretion also affords protection against proteolytic degradation of gene products by naturally occurring protease enzymes. Microorganisms are notorious for producing enzymes which rapidly digest unprotected foreign polypeptides. The present method for secretion circumvents this problem by providing a means for removing susceptible polypeptides from the host cell before proteolytic degradation can occur. In addition, host cells also are protected from the toxic effects of a given gene product because secretion prevents the deleterious effects, including possible cell death, associated with intracellular build-up.

*Bacillus subtilis* MI112/pOW430, *B. subtilis* MI112/pOW440 and *E. coli* K12 JA221/pNM587.4—4, as sources of plasmids pOW430, pOW440 and pNM587.4—4, respectively, can be cultured in a number of ways using any of several different media. Carbohydrate sources which are preferred in a culture medium include, for example, molasses, glucose, dextrin, and glycerol, and nitrogen sources include, for example, soy flour, amino acid mixtures, and peptones. Nutrient inorganic salts are incorporated also and include the customary salts capable of yielding sodium, potassium, ammonium, calcium, phosphate, chloride, sulfate, and like ions. As is necessary for the growth and development of other microorganisms, essential trace elements are added also. Such trace elements commonly are supplied as impurities incidental to the addition of other constituents of the medium.

*Bacillus subtilis* MI112/pOW430 and *B. subtilis* MI112/pOW440 are grown under aerobic culture conditions over a relatively wide pH range of about 5 to 8.5 at temperatures ranging from about 25° to 45°C. For production of plasmids pOW430 and pOW440 in the greatest quantities, however, it is desirable to start with a culture medium at a pH of about 7 and maintain a culture temperature of about 37°C. Culturing *Bacillus subtilis* MI112/pOW430 and *B. subtilis* MI112/pOW440 under these conditions results in a reservoir of cells from which these plasmids are isolated conveniently by techniques well known in the art.

*E. coli* K12 JA221/pNM587.4—4 is grown under aerobic culture conditions over a relatively wide pH range of about 6.5 to 8 at temperatures ranging from about 25° to 40°C. It is desirable to start with a culture medium at a pH of about 7.2 and maintain a culture temperature of about 37°C. Culturing the *E. coli* cells, under these conditions, results in a reservoir of cells from which the plasmids are isolated respectively by techniques well known in the art.

The following non-limiting examples are provided to further illustrate and detail the invention. Both an explanation of and the actual procedures for constructing the invention are described where appropriate.

## Example 1
### Culture of *Bacillus subtilis* MI112/pOW440

A vegetative culture of *Bacillus subtilis* MI112/pOW440 (NRRL B—15887) was prepared conventionally by inoculating sterile Penassay broth (Difco) containing 20 µg/ml of chloramphenicol with the above-specified strain and growing the resultant starter culture at 37°C with vigorous aeration for about 12 hours. About 2—5% of the volume of the starter culture then is added to fresh sterile Penassay broth and grown at 37°C. until the culture is turbid (about 300—500 Klett units on a Klett Summerson Colorimeter, Klett Mfg. Co., Inc. New York, New York, with filter #60). Cell extracts and medium were assayed for nuclease activity in substantial accordance with the procedure of Cuatrecasas et al., 1967, *J. Biol. Chem.* 242:1541, except that all assays were performed at ambient temperature. Staphylococcal nuclease is produced in high amounts and can be isolated conventionally from the cells and also the culture medium.

## Example 2
### Construction of Plasmid pOW448 and *Bacillus subtilis* MI112/pOW448
A. Construction of Plasmid pOW650 and *E. coli* JA221/pOW650
   1. Isolation of Plasmid pOW440

About 10 g (wet wgt) of *Bacillus subtilis* MI112/pOW440 cells (grown in Example 1) were harvested by centrifugation (10 minutes, 4°C, 10,000 rpm), washed in about 50 ml TES (10 mM Tris (pH 8), 10 mM NaCl, 1 mM EDTA) and finally collected again by centrifugation. About 20 ml of TE buffer (containing 25% sucrose) were added followed by 10 mg of lysozyme in 250 µl water. After the mixture was incubated at 37°C for about 30 minutes, about 100 units of RNase were added. The resultant mixture was again incubated at 37°C for 30 minutes and, upon being made 1% and 1 M with respect to SDS (sodium dodecyl sulfate) and sodium chloride, respectively, the mixture was cooled in an ice bath for about 3 hours. After the lysate was centrifuged (30 minutes, 4°C, 19,000 rpm), the supernatant was adjusted to 31.8 ml with TE and then 28.7 g of cesium chloride and .4 ml (10 mg/ml) of ethidium bromide were added. A cesium chloride gradient was

EP 0 176 320 B1

established by centrifuging at 49,500 rpm for 16 hours in a VTi50 rotor (Beckman Instruments). The plasmid band was collected and centrifuged at 55,000 rpm in a VTi80 rotor (Beckman Instruments) for 16 hours, then collected again, extracted until all color had disappeared (and then extracted once more) with equal volumes of isoamyl alcohol, dialyzed against dilute TE, ethanol precipitated, and resuspended in 400 μl of TE. The resultant plasmid pOW440 DNA was stored at 4°C for future use.

2. *Xho*II Digestion of Plasmid pOW440 and Isolation of the ~.65 kb Fragment

About 20 μl (20 μg) of plasmid pOW440 in water, 1 μl DTT (10 mM Dithiothreitol), 1 μl (10 μg/ml) BSA (bovine serum albumin) 20 μl water, 4 μl (6 units) *Xho*II restriction enzyme and 5 μl 10X reaction mix* were incubated at 37°C for about 1 hour. The reaction was terminated by incubation at 65°C for 10 minutes and then the reaction mixture was cooled on ice, extracted with each of phenol and chloroform:isoamyl alcohol (24:1) and then ethanol precipitated. The desired ~.65 kb restriction fragments were separated conventionally and isolated by agarose gel electrophoresis (Maniatis *et al.*, 1982, Molecular Cloning, Cold Spring Harbor Laboratory, Cold Spring Harbor, New York). The desired ~.65 kb fragments were dissolved in about 10 μl of water.

* Reaction mix for *Xho*II restriction enzyme was prepared with the following composition.
100 mM Tris-HCl, pH8
100 mM $MgCl_2$
.1% Triton X—100

3. *Bam*HI-*Bg*II Digestion of Plasmid pKC7 (ATCC 37084)

The desired digestion was carried out in substantial accordance with the teaching of Example 2A—2 except that *Bam*HI and *Bg*II restriction enzymes, rather than *Xho*II restriction enzyme, were used. After ethanol precipitation, the digest was dissolved in 10 μl water and used without further purification.

4. Ligation and construction of *E. coli* K12 JA221/pOW650

About 4 μl (3 μg) of the ~.65 kb *Xho*II fragment of plasmid pOW440, 2 μl (1 μg) of the *Bam*HI-*Bg*II digest of plasmid pKC7, 10 μl water, 2 μl (10 mM) ATP, 1 μl DTT, 2 μl ligation mix** and 1 μl T4 DNA ligase (4 units) were incubated at 16°C for about 16 hours. The reaction was terminated by incubation at 65°C for 10 minutes and then, after cooling on ice, the resultant ligated mixture was used to transform *E. coli* K12 JA221 in substantial accordance with the transformation procedure of Lederburg and Cohen, 1974, *J. Bacteriology* 119:1072, on TY agar (10 g/L tryptone, 5 g/L yeast extract, 5 g/L NaCl, pH 7.2, 15 g/L Agar) containing 80 μg/ml of antibiotic ampicillin. The resultant transformants were cultured conventionally and identified and the desired transformants used for the subsequent production and isolation of plasmid pOW650.

** Ligation mix was prepared with the following composition.
500 mM Tris-HCl, pH 7.8
100 mM $MgCl_2$

B. Construction of Plasmid pOW340 and *E. coli* K12 JA221/pOW340
1. Construction of the DNA Sequence

```
5' GAT CCA ACA GTA TAT AGT GCA ACT TTC GTT AAC CAA CAC TTG T 3'
    || ||| ||| ||| ||| ||| ||| ||| ||| ||| ||| ||| |||
3'     GT TGT CAT ATA TCA CGT TGA AAG CAA TTG GTT GTG AAC   5'
```

wherein
A is deoxyadenyl,
G is deoxyguanyl,
C is deoxycytosyl and
Y is thymidyl.

The desired fragment was constructed using an automated phosphite triester method. Although any DNA synthesizer can be used, the DNA Synthesizer 380A of Applied Biosystems, Foster City, California is preferred. Those skilled in the art will recognize that the above sequence also can be synthesized conventionally in accordance with the procedure of Itakura *et al.*, 1977, *Science* 198:1056 and Crea *et al.*, 1978, *proc. Nat. Acad. Sci. USA* 75:5765. In addition, an especially preferred synthetic method is disclosed in Hsiung *et al.*, 1983, *Nucleic Acid Research* 11:3227 and Narang *et al.*, 1980, *Methods in Enzymology* 68:90. The desired fragment was dissolved in 10 mM Tris, pH 7.8 and stored at −20°C for future use.

2. *Hph*I-*Xho*II Digestion of Plasmid pNM587.4—4 and Isolation of the ~.27 kb Fragment
a. Isolation of Plasmid pNM587.4—4
The bacterium *E. coli* K12 JA221/pNM587.4—4 (NRRL B—15812) was cultured in TY broth (the same as

7

EP 0 176 320 B1

TY agar except without the agar) with 100 µg/ml of antibiotic ampicillin according to conventional-microbiological procedures. After 18 hours incubation, about .5 ml of the culture was transferred to a 1.5 ml Eppendorf tube and centrifuged for about 15 seconds. Unless otherwise indicated, all the manipulations were done at ambient temperature. The resultant supernatant was carefully removed with a fine-tip aspirator and the cell pellet suspended in about 100 µl of freshly prepared lysozyme solution which contained 2 mg/ml lysozyme, 50 mM glucose, 10 mM EDTA (ethylene diaminetetracetate) and 25 mM Tris-HCl (pH 8). After incubation at 0°C for 30 minutes, about 200 µl of alkaline SDS (sodium dodecyl sulfate) solution (.2N NaOH, 1% SDS) were added and then the tube was gently vortexed and maintained at 0°C for 5 minutes. Next, about 150 µl of 3M sodium acetate (prepared by dissolving 3 moles of sodium acetate in a minimum of water, adjusting the pH to 4.8 with glacial acetic acid and then adjusting the volume to 1 L) were added. A DNA clot formed after the contents of the tube were mixed gently for a few seconds by inversion.

The tube was maintained at 0°C for 60 minutes and then centrifuged for 5 minutes to yield an almost clear supernatant. About .4 ml of the supernatant was transferred to a second centrifuge tube to which 1 ml of cold ethanol was added. After the tube was held at −20°C for 30 minutes, the resultant precipitate was collected by centrifugation and the supernatant removed by aspiration. The DNA pellet was dissolved in 200 µl of .1M sodium acetate/.05 N Tris-HCl (pH 8) and was reprecipitated by the addition of 2 volumes of cold ethanol. After 10 minutes at −20°C, the precipitate was collected by centrifugation and constituted the desired plasmid pNM587.4—4 DNA.

b. *Hph*I-*Xho*II Digestion of Plasmid pNM587.4—4 and Isolation of ~.27 kb Fragment
About 20 µl (20 µg) of plasmid pNM587.4—4 in water 2 µl DTT, 1 µl (1000 µg/ml) BSA, 20 µl wate, 4 µl (8 units) of each of *Hph*I and *Xho*II restriction enzymes and 500 µl 10X reaction mix* were incubated at 37°C for about 1 hour. The reaction was terminated by incubation at 65°C for 10 minutes and then the resultant mixture was cooled on ice, extracted with each of phenol and chloroform:isoamyl alcohol (24:1) and then ethanol precipitated. The desired ∿.27 kb *Hph*I-*Xho*II restriction fragments were separated conventionally and isolated by acrylamide gel electrophoresis (Maniatis et al., 1982) and then dissolved in about 30 µl of water.

* Reaction mix for *Hph*I-*Xho*II restriction enzymes was prepared with the following composition:
100 mM KCl
100 mM Tris-HCl, pH 7.5
100 mM MgCl₂

c. *Bam*HI digestion of plasmid pUC8
The desired digestion of plasmid pUC8, commercially available from Bethesda Research Laboratory, Gaithersburg, Maryland, was carried out in substantial accordance with the teaching of Example 2A—2 except that *Bam*HI restriction enzyme and reaction mix**, rather than *Xho*II restriction enzyme and reaction mix, were used and except that after ethanol precipitation, the digest was dissolved in 10 µl water and used without further purification.

** Reaction mix for *Bam*HI restriction enzymes was prepared with the following composition:
500 mM NaCl
500 mM Tris-HCl, pH 8
100 mM MgCl₂

d. Ligation and Construction of *E. coli* K12 JA221/pOW340
About 2 µl (.5 µg) of the DNA sequence of Example 2B—1, 3 µl (.8 µg) of the ~.27 kb *Hph*I-*Xho*II fragment of plasmid pNM587.4—4 and 2 µl (1 µg) of the *Bam*HI digest of pUC8 were ligated and *E. coli* K12 JA221 transformed in substantial accordance with the teaching of Example 2A—4. The resultant transformants were cultured conventionally and identified and the desired transformants used for the subsequent production and isolation of plasmid pOW340.

C. Construction of Plasmid pOW341 and *E. coli* K12 JA221/pOW341
1. *Bgl*II Digestion of Plasmid pOW650
The desired digestion of plasmid pOW650 was carried out in substantial accordance with the teaching of Example 2A—2 except that *Bgl*II restriction enzyme and reaction mix***, rather than *Xho*II restriction enzyme and reaction mix, were used and except that after ethanol precipitation, the digest was dissolved in 10 µl water and used without further purification.

*** Reaction mix for *Bgl*II restriction enzyme was prepared with the following composition.
500 mM NaCl
60 mM Tris-HCl, pH 7.5
60 mM MgCl₂

8

2. *Bam*HI Digestion of Plasmid pOW340

The desired digestion was carried out in substantial accordance with the teaching of Example 2A—2 except that *Bam*HI restriction enzyme and reaction mix, rather than *Xho*II restriction enzyme and reaction mix, were used and except that after ethanol precipitation, the digest was dissolved in 10 μl water and used without further purification.

3. Ligation and Construction of *E. coli* K12 JA221/pOW341

About 2 μl (.8 μg) of the *Bgl*II digest of plasmid pOW650 and 2 μl (1 μg) of the *Bam*HI digest of plasmid pOW340 were ligated and *E. coli* K12 JA221 transformed in substantial accordance with the teaching of Example 2A—4. The resultant transformants were cultured conventionally and screened for the desired plasmid pOW341. Plasmids with the correct orientation of fragments were identified by a *Hpa*I digestion followed by electrophoresis in an 8% acrylamide gel and analysis of fragments. A restriction site map of plasmid pOW341 is presented in Figure 3 of the accompanying drawing. A desired *E. coli* K12 JA221/pOW341 transformant was cultured conventionally and used for subsequent production and isolation of plasmid pOW341.

D. Construction of Plasmid pOW448 and *E. coli* K12 JA221/pOW448

1. *Eco*RI Digestion of Plasmid pOW341

The desired digestion was carried out in substantial accordance with the teaching of Example 2A—2 except that *Eco*RI restriction enzyme and reaction mix*, rather than *Xho*II restriction enzyme and reaction mix, were used and.except that after ethanol precipitation, the digest was dissolved in 10 μl water and used without further purification.

* Reaction mix for *Eco*RI restriction enzyme was prepared with the following composition.
500 mM NaCl
500 mM Tris-HCl, pH 8
60 mM MgCl$_2$

2. *Eco*RI Digestion of Plasmid pOW430

Plasmid pOW430 was isolated from a vegetative culture of *Bacillus subtilis* MI112/pOW430 (NRRL B—15833) in substantial accordance with the teaching of Example 1. Plasmid pOW430 was then digested in substantial accordance with the teaching of Example 2A—2 except that *Eco*RI restriction enzyme and reaction mix, rather than *Xho*II restriction enzyme and reaction mix, were used and except that after ethanol precipitation, the digest was dissolved in 10 μl water and used without further purification.

3. Ligation and Construction of *E. coli* K12 JA221/pOW448

About 2 μl (1 μg) of each of the *Eco*RI digests of plasmids pOW341 and pOW430 were ligated and *E. coli* K12 JA221 transformed in substantial accordance with the teaching of Example 2A—4. The resultant transformants were cultured conventionally and screened for the desired plasmid pOW448. Transformants containing plasmids with both of the fragments were identified by selecting for ampicillin and chloramphenicol resistance. A desired *E. coli* K12 JA221/pOW448 transformant was cultured conventionally and used for the subsequent production and isolation of plasmid pOW448. A restriction site map of plasmid pOW448 is presented in Figure 2 of the accompanying drawings.

E. Construction of *Bacillus subtilis* MI112/pOW448

*Bacillus subtilis* MI112 can be obtained by conventionally culturing *B. subtilis* MI112/pOW430 (NRRL B—15833) in the absence of chloramphenicol. The *B. subtilis* MI112/pOW430 cells spontaneously lose the pOW430 plasmid under the aforementioned culture conditions thus generating the desired chloramphenicol sensitive *B. subtilis* MI112 strain. Those skilled in the art will recognize and understand that sensitivity to chloramphenicol can be employed for testing and insuring that only *B. subtilis* MI112 cells that lack the plasmid are selected and used in the *Bacillus* transformation procedures disclosed.

About 50 ml of sterile PAB (Penassay broth) was inoculated with *Bacillus subtilis* MI112 and incubated at 37°C until a cell density of $2 \times 10^8$ cells/ml was reached. The cells were then protoplasted using sterile technique, by pelleting and then resuspending the cells in about 5 ml of SMMP (equal volumes of each of 4x PAB and a solution comprising 1M sucrose, .04 M maleic acid, and .04 M MgCl$_2$, pH adjusted to 6.5 with NaOH). Next, about 250 μl of lysozyme (20 mg/ml in SMM [0.5 M sucrose, .02 M maleic acid, and .02 M MgCl$_2$, pH adjusted to 6.5 with NaOH]) were added using filter sterilization. The cells were incubated with gentle shaking at 37°C for about 2 hours. The resultant protoplasts were pelleted, washed with 5 ml SMMP, and then resuspended in 5 ml SMMP. Following centrifugation (25°C, 12 minutes, 2,600 rpm), about .1 ml of protoplasts was transformed by adding about 20 μl of a 1:1 mixture comprising plasmid pOW448 DNA and 2X SMM. About 1.5 ml of PEG solution (40 g PEG 6000 [polyethyleneglycol], 50 ml 2X SMM, and water to 100 ml) then were added followed by about 5 ml of SMMP after about 2 minutes. Next, the protoplasts were pelleted, suspended in 1 ml of SMMP, and incubated at 30°C with gentle shaking for about 2 hours. Aliquots of the resultant suspension were plated on chloramphenicol-containing DM3 regeneration medium which per liter had the following composition:

9

| 91 g | D-mannitol in 555 ml deionized water containing 12 g agar |
|------|------|
| 10% | Casamino acids 50 ml |
| 10% | Yeast extract 50 ml |
| 20% | Glucose 25 ml |
| 5% | Dipotassium phosphate 100 ml |
| 1M | $MgCl_2$ 20 ml |
| 10% | Gelatin |

The D-mannitol, casamino acids and yeast extract were autoclaved together. The gelatin was added immediately after autoclaving and the remaining ingredients were added after the mixture had cooled. The medium had a final antibiotic chloramphenicol concentration of 10 µg/ml.

A chloramphenicol resistant colony was selected as the desired *Bacillus subtilis* MI112/pOW448 strain. The strain was cultured and the identity further confirmed by conventional restriction enzyme and agarose gel electrophoretic analysis (Maniatis *et al.,* 1982), of the constitutive plasmid. The desired *Bacillus subtilis* MI112/pOW448 transformants were shown to express human proinsulin and also to secrete the proinsulin product into the culture medium. The presence of human proinsulin both intracellularly and in the medium was determined conventionally by radioimmunoassay.

Example 3
Derivation of *Bacillus subtilis* SR22 and *B. subtilis* SR22/pOW448

*Bacillus subtilis* SR22 can be derived conventionally from *B. subtilis* SR22/pOW440, a strain deposited and made part of the permanent culture collection of the Northern Regional Research Laboratory, Peoria, Illinois, under the accession number NRRL B—15893, by conventionally culturing the organism in the absence of chloramphenicol. The *B. subtilis* SR22/pOW440 cells spontaneously lose the pOW440 plasmid under the aforementioned culture condition thus generating the desired chloramphenicol sensitive *B. subtilis* SR22 strain. Those skilled in the art will recognize and understand that sensitivity to chloramphenicol can be employed for testing and insuring that only *B. subtilis* SR22 cells lacking the plasmid are selected and used for generating plasmid pOW448 transformants in accordance with the previous teachings.

## Claims

1. A method for expressing a functional polypeptide in *Bacillus* which comprises:
    a) transforming a *Bacillus* host cell with a recombinant DNA expression vector which is selectable and capable of replication in said host cell, the vector comprising
        1) the transcriptional and translational activating sequence of the *Staphylococcus aureus* nuclease gene and
        2) a DNA sequence that codes for a functional polypeptide; and
    b) culturing the transformed cell under conditions suitable for expression of said polypeptide,
subject to the limitation 1) that the functional polypeptide sequence and the transcriptional and translational activating sequence are immediately adjacent, in translational reading frame and positioned for expression of said functional polypeptide and 2) that the functional polypeptide sequence is exclusive of the nucleotide triplet that codes for the N-terminal amino acid of the functional polypeptide when said amino acid is methionine.

2. A method as claimed in Claim 1 in which
    a) the recombinant DNA expression vector further comprises a signal peptide coding sequence, and
    b) the transformed cells are cultured under conditions suitable for expression and secretion of said functional polypeptide,
subject to the limitation 1) that the signal peptide coding sequence is exclusive of the nucleotide triplet that codes for the N-terminal amino acid of the encoded signal peptide when said amino acid is methionine and that the signal peptide coding sequence is positioned for expression immediately adjacent to, downstream of and in translational reading frame with said transcriptional and translational activating sequence, 2) that the nucleotide triplet coding for the C-terminus of the signal peptide encoded by said signal peptide coding sequence is immediately adjacent to, upstream of and in translational reading frame with the functional polypeptide sequence, and 3) that the functional polypeptide sequence is inclusive of the nucleotide triplet that codes for the N-terminal amino acid of said functional polypeptide.

3. A method as claimed in claim 1 or 2 in which the functional polypeptide sequence codes for human proinsulin, human insulin A-chain, human insulin B-chain, human pre-proinsulin, human growth hormone, bovine growth hormone, porcine growth hormone, growth hormone releasing factor, interferon, interleukin II, an enzyme, or a hormone.

4. A method as claimed in Claim 3 in which the functional polypeptide sequence is that for human proinsulin.

5. A recombinant DNA expression vector used in the method of Claim 1 or 2.

6. A recombinant DNA expression vector as claimed in Claim 5 which is a plasmid.

7. A recombinant DNA expression vector for use in a method for expressing a functional polypeptide in *Bacillus* which comprises:

a) transforming a *Bacillus* host cell with a recombinant DNA expression vector which is selectable and capable of replication in said host cell, the vector comprising

1) the transcriptional and translational activating sequence of the *Staphylococcus aureus* nuclease gene and

2) a DNA sequence that codes for a functional polypeptide; and

b) culturing the transformed cell under conditions suitable for expression of said polypeptide,

subject to the limitation 1) that the functional polypeptide sequence and the transcriptional and translational activating sequence are immediately adjacent, in translational reading frame and positioned for expression of said functional polypeptide and 2) that the functional polypeptide sequence is exclusive of the nucleotide triplet that codes for the N-terminal amino acid of the functional polypeptide when said amino acid is methionine;

in which the functional polypeptide sequence codes for human growth hormone, human insulin, human insulin A-chain, human insulin B-chain, human pre-proinsulin, human proinsulin, bovine growth hormone, porcine growth hormone, interferon, growth hormone releasing factor, interleukin II, an enzyme or a hormone.

8. A recombinant DNA expression vector as claimed in Claim 7 in which the functional polypeptide sequence codes for human proinsulin.

9. The plasmid shown in Figure 1 named pOW440 obtainable from *Bacillus subtilis* MI112/pOW440 (NRRL B—15887). .

10. The plasmid shown in Figure 2 named pOE448 isolated from *E. coli* K12 JA221/pOW448 produced by transforming *E. coli* K12 JA221 with the ligated *Eco*RI digests of plasmids pOW341 and pOW430 defined below.

11. Plasmid pOW430 obtainable from *Bacillus subtilis* MI112/pOW430 (NRRL B—15833).

12. Plasmid pOW650 obtained by ligation of the ~.65 kb *Xho*II fragment of plasmid pOW440 defined in claim 9 and the *Bam*HI-*Bgl*II digest of plasmid pKC7 (from ATCC 37084).

13. Plasmid pOW50 obtained by filling in both the *Bam*HI site of plasmid pBR322 and the Staphylococcal nuclease gene-containing ~1.4 kb *Hpa*II fragment from plasmid pFOG301, by use of Klenow enzyme, and then ligating the resultant flush-ended fragments.

14. Plasmid pOW340 obtained by ligating the appropriate DNA sequence, the ~.27 kb *Hph*I-*Xho*II fragment of plasmid pNM587.4—4 (obtainable from *E. coli* JA221/pNM587.4—4, NRRL B—15812) and the *Bam*HI digest of pUC8.

15. The plasmid shown in Figure 3 named pOW341 obtainable from *E. coli* K12 JA221/pOW341, produced by ligating the *Bgl*II digest of plasmid pOW650 defined in claim 12 and the *Bam*HI digest of plasmid pOW340 defined in claim 14 and transforming *E. coli* K12 JA221.

16. A transformed host cell for use in the method of claim 1 or 2 comprising a DNA expression vector as claimed in any one of claims 5 to 15.

17. A host cell transformed by a DNA expression vector as claimed in any one of claims 5 to 15.

18. A host cell of claim 17 which is *Bacillus* or *E. coli*.

19. A host cell of claim 18 which is *Bacillus subtilis*.

20. An *E. coli* host cell transformed by a plasmid claimed in any one of claims 11 to 15.

21. A transformed host cell selected from the group of *Bacillus subtilis* MI112 transformed by plasmid pOW440 defined in claim 9, *Bacillus subtilis* MI112 transformed by plasmid pOW430 defined in claim 11, *Bacillus subtilis* SR22 transformed by plasmid pOW448 defined in claim 10, *Bacillus subtilis* MI112 transformed by plasmid pOW448, *Bacillus subtilis* SR22 transformed by plasmid pOW440, *E. coli* K12 JA221 transformed by plasmid pOW448, *E. coli* K12 JA221 transformed by plasmid pOW341 defined in claim 15, *E. coli* K12 JA221 transformed by plasmid pOW340 defined in claim 14, *E. coli* K12 JA221 transformed by plasmid pOW650 defined in claim 12, or *Bacillus subtilis* MI112 transformed by plasmid pOW430.

## Patentansprüche

1. Verfahren zur Expression eines funktionellen Polypeptids in Bacillus, umfassend:

a) die Transformation einer Bacillus-Wirtszelle mit einem rekombinanten DNA-Expressionsvektor, der selektierbar und zur Replikation in der Wirtszelle fähig ist, wobei der Vektor folgendes umfasst:

1) die transkriptionale und translationale Aktivierungssequenz des Staphylococcus aureus-Nuclease-Gens und

2) eine DNA-Sequenz, die für ein funktionelles Polypeptid kodiert; und

b) die Züchtung der transformierten Zelle unter für die Expression des Polypeptids geeigneten Bedingungen,

mit der Beschränkung, 1) dass die funktionelle Polypeptidsequenz und die transkriptionale und translationale Aktivierungssequenz unmittelbar benachbart, im translationalen Leserahmen und für die Expression des funktionellen Polypeptids positioniert sind und 2) dass die funktionelle Polypeptidsequenz das Nucleotid-Triplett nicht einschliesst, das für die N-terminale Aminosäure des funktionellen Polypeptids kodiert, wenn diese Aminosäure Methionin ist.

2. Verfahren nach Anspruch 1, bei dem

a) der rekombinante DNA-Expressionsvektor ferner eine Signalpeptid-Kodierungssequenz umfasst und

b) die transformierten Zellen unter Bedingungen gezüchtet werden, die für die Expression und Sekretion des funktionellen Polypeptids geeignet sind, mit der Beschränkung, 1) dass die Signalpeptid-Kodierungssequenz das Nucleotid-Triplett, das für die N-terminale Aminosäure des kodierten Signalpeptids kodiert, nicht einschliesst, wenn die Aminosäure Methionin ist, und dass die Signalpeptid-Kodierungssequenz für die Expression unmittelbar benachbart zur transkriptionalen und translationalen Aktivierungssequenz, stromabwärts davon und im translationalen Leserahmen damit positioniert ist, 2) dass das Nucleotid-Triplett, das für das C-Ende des durch die Signalpeptid-Kodierungssequenz kodierten Signalpeptids kodiert, sich unmittelbar neben der funktionellen Polypeptidsequenz, stromaufwärts davon und im translationalen Leserahmen damit befindet und 3) dass die funktionelle Polypeptidsequenz das Nucleotid-Triplett einschliesst, das für die N-terminale Aminosäure des funktionellen Polypeptids kodiert.

3. Verfahren nach Anspruch 1 oder 2, bei dem die funktionelle Polypeptidsequenz für humanes Proinsulin, humane Insulin A-Kette, humane Insulin B-Kette, humanes Präproinsulin, humanes Wachstumshormon, Rinderwachstumshormon, Schweinewachstumshormon, Wachstumshormon-Freisetzungsfaktor, Interferon, Interleukin II, ein Enzym oder ein Hormon kodiert.

4. Verfahren nach Anspruch 3, bei dem es sich bei der funktionellen Polypeptidsequenz um die Sequenz für humanes Proinsulin handelt.

5. Rekombinanter DNA-Expressionsvektor, verwendet im Verfahren von Anspruch 1 oder 2.

6. Rekombinanter DNA-Expressionsvektor nach Anspruch 5, bei dem es sich um ein Plasmid handelt.

7. Rekombinanter DNA-Expressionsvektor zur Verwendung in einem Verfahren zur Expression eines funktionellen Polypeptids in Bacillus, umfassend:

a) die Transformation einer Bacillus-Wirtszelle mit einem rekombinanten DNA-Expressionsvektor, der selektierbar und zur Replikation in der Wirtszelle fähig ist, wobei der Vektor folgendes umfasst:

1) die transkriptionale und translationale Aktivierungssequenz des Staphylococcus aureus-Nuclease-Gens und

2) eine DNA-Sequenz, die für ein funktionelles Polypeptid kodiert; und

b) die Züchtung der transformierten Zelle unter für die Expression des Polypeptids geeigneten Bedingungen,

mit der Beschränkung, 1) dass die funktionelle Polypeptidsequenz und die transkriptionale und translationale Aktivierungssequenz unmittelbar benachbart, im translationalen Leserahmen und für die Expression des funktionellen Polypeptids positioniert sind und 2) dass die funktionelle Polypeptidsequenz das Nucleotid-Triplett nicht einschliesst, das für die N-terminale Aminosäure des funktionellen Polypeptids kodiert, wenn diese Aminosäure Methionin ist; wobei die funktionelle Polypeptidsequenz für humanes Wachstumshormon, humanes Insulin, humane Insulin A-Kette, humane Insulin B-Kette, humanes Präproinsulin, humanes Proinsulin, Rinderwachstumshormon, Schweinewachstumshormon, Interferon, Wachstumshormon-Freisetzungsfaktor, Interleukin II, ein Enzym oder ein Hormon kodiert.

8. Rekombinanter DNA-Expressionsvektor nach Anspruch 7, bei dem die funktionelle Polypeptidsequenz für humanes Proinsulin kodiert.

9. Plasmid gemäss Fig. 1, mit der Bezeichnung pOW440, enhältlich aus Bacillus subtilis MI112/pOW440 (NRRL B—15887).

10. Plasmid gemäss Fig. 2, mit der Bezeichnung pOW448, isoliert aus E. coli K12 JA221/pOW448, hergestellt durch Transformieren von E. coli K12 JA221 mit den verknüpften EcoRI-Verdauungsprodukten der nachstehend definierten Plasmide pOW341 und pOW430.

11. Plasmid pOW430, erhältlich aus Bacillus subtilis MI112/pOW430 (NRRL B—15833).

12. Plasmid pOW650, erhalten durch Verknüpfung des ~0,65 kb-XholI-Fragments des Plasmids pOW440 gemäss Anspruch 9 und des BamHI-BglII-Verdauungsprodukts des Plasmids pKC7 (aus ATCC 37084).

13. Plasmid pOW50, erhalten durch Auffüllen der BamHI-Stelle des Plasmids pBR322 und des Staphylococcen-Nuclease-Gens mit einem Gehalt an dem ~1,4 kb-HpaII-Fragment aus dem Plasmid pFOG301 unter Verwendung von Klenow-Enzym und durch anschliessende Verknüpfung der erhaltenen stumpfendigen Fragmente.

14. Plasmid pOW340, erhalten durch Verknüpfung der entsprechenden DNA-Sequenz, des ~0,27 kb-HphI-XholI-Fragments des Plasmids pNM587.4—4 (erhältlich aus E. Coli JA221/pNM587.4—4, NRRI B—15812) und des BamHI-Verdauungsprodukts von pUC8.

15. Plasmid gemäss Fig. 3 mit der Bezeichnung pOW341, erhältlich aus E. coli K12 JA221/pOW341, hergestellt durch Verknüpfen des BglII-Verdauungsprodukts des Plasmids pOW650 gemäss Anspruch 12 und des BamII-Verdauungsprodukts des Plasmids pOW430 gemäss Anspruch 14 und durch Transformation von E. coli K12 JA221.

16. Transformierte Wirtszelle zur Verwendung im Verfahren von Anspruch 1 oder 2, enthaltend einen DNA-Expressionsvektor nach einem der Ansprüche 5 bis 15.

17. Wirtszelle, transformiert durch einen DNA-Expressionsvektor nach einem der Ansprüche 5 bis 15.

18. Wirtszelle nach Anspruch 17, wobei es sich um Bacillus oder E. coli handelt.

19. Wirtszelle nach Anspruch 17, wobei es sich um Bacillus subtilis handelt.

20. E. coli-Wirtszelle, transformiert durch ein Plasmid nach einem der Ansprüche 11 bis 15.

21. Transformierte Wirtszelle, ausgewählt aus folgender Gruppe: Plasmid pOW440 nach Anspruch 9, Bacillus subtilis MI112, transformiert durch das Plasmid pOW430 nach Anspruch 11, Bacillus subtilis SR22, transformiert durch das Plasmid pOW448 nach Anspruch 10, Bacillus subtilis MI112 transformiert durch das Plasmid pOW448, Bacillus subtilis SR22, transformiert durch das Plasmid pOW440, E. coli K12 JA221, transformiert durch das Plasmid pOW448, E. coli K12 JA221, transformiert durch das Plasmid pOW341 nach Anspruch 15, E. coli K12 JA221, transformiert durch das Plasmid pOW340 nach Anspruch 14, E. coli K12 JA221, transformiert durch das Plasmid pOW650 nach Anspruch 12 oder Bacillus subtilis durch das Plasmid pOW650 nach Anspruch 12 oder Bacillus subtilis MI112, transformiert durch das Plasmid pOW430.

**Revendications**

1. Procédé pour l'expression d'un polypeptide fonctionnel dans Bacillus, caractérisé en ce qu'il comprend les étapes qui consistent à:

a) transformer une cellule hôte de Bacillus avec un vecteur d'expression d'ADN recombinant pouvant être sélectionné et capable de réplication dans cette cellule hôte, le vecteur comprenant

1) la séquence activatrice transcriptionnelle et translationnelle du gène de nucléase de Staphylococcus aureus, et

2) une séquence d'ADN codant un polypeptide fonctionnel; et

b) cultiver la cellule transformée dans des conditions appropriées pour l'expression de ce polypeptide, avec les restrictions suivantes: 1) la séquence de polypeptides fonctionnels et la séquence activatrice transcriptionnelle et translationnelle sont immédiatement adjacentes, dans le cadre de lecture translationnelle, tout en étant localisées pour l'expression de ce polypeptide fonctionnel et 2) la séquence de polypeptides fonctionnels est exclusive pour le triplet de nucléotides codant l'amino-acide N-terminal du polypeptide fonctionnel lorsque cet amino-acide est la méthionine.

2. Procédé selon la revendication 1, dans lequel:

a) le vecteur d'expression d'ADN recombinant comprend, en outre, une séquence de codage de peptides signaux, et

b) les cellules transformées sont cultivées dans des conditions appropriées pour l'expression et la sécrétion de ce polypeptide fonctionnel, avec cette restriction que 1) la séquence de codage des peptides signaux est exclusive pour le triplet nucléotide codant l'amino-acide N-terminal du peptide signal encodé lorsque cet amino-acide est la méthionine et que la séquence de codage des peptides signaux est localisée pour l'expression à proximité immédiate de, en aval de et dans le cadre de lecture translationnelle avec cette séquence activatrice transcriptionnelle et translationnelle, 2) le triplet de nucléotides codant la terminaison C du peptide signal encodé par la séquence de codage des peptides signaux est immédiatement adjacent à, en aval de et dans le cadre de lecture translationnelle avec la séquence de polypeptides fonctionnels, et 3) la séquence des polypeptides fonctionnels fait partie du triplet de nucléotides codant l'amino-acide N-terminal du polypeptide fonctionnel.

3. Procédé selon la revendication 1 ou 2, dans lequel la séquence de polypeptides fonctionnels code la pro-insuline humaine, la chaîne A d'insuline humaine, la chaîne B d'insuline humaine, la pré-pro-insuline humaine, l'hormone de croissance humaine, l'hormone de croissance bovine, l'hormone de croissance porcine, le facteur libérant l'hormone de croissance, l'interféron, l'interleucine II, une enzyme, ou une hormone.

4. Procédé selon la revendication 3, dans lequel la séquence de polypeptides fonctionnels est celle prévue pour la pro-insuline humaine.

5. Vecteur d'expression d'ADN recombinant utilisé dans le procédé selon les revendication 1 ou 2.

6. Vecteur d'expression d'ADN recombinant selon la revendication 5, caractérisé en ce qu'il est un plasmide.

7. Vecteur d'expression d'ADN recombinant en vue de l'utiliser dans un procédé pour l'expression d'un polypeptide fonctionnel dans Bacillus, caractérisé en ce qu'il comprend les étapes qui consistent à:

a) transformer une cellule hôte de Bacillus avec un vecteur d'expression d'ADN recombinant qui peut être sélectionné et est capable de réplication dans la cellule hôte précitée, ce vecteur comprenant:

1) la séquence activatrice transcriptionnelle et translationnelle du gène de nucléase de Staphylococcus aureus, et

2) une séquence d'ADN codant un polypeptide fonctionnel; et

b) cultiver la cellule transformée dans des conditions appropriées pour l'expression de ce polypeptide, avec les restrictions suivantes: 1) la séquence de polypeptides fonctionnels et la séquence activatrice transcriptionnelle et translationnelle sont immédiatement adjacentes, dans le cadre de lecture translationnelle, tout en étant localisées pour l'expression de ce polypeptide fonctionnel et, 2) la séquence de polypeptides fonctionnels est exclusive pour le triplet de nucléotides codant l'amino-acide N-terminal du polypeptide fonctionnel lorsque cet amino-acide est la méthionine;

tandis que la séquence de polypeptides fonctionnels code l'hormone de croissance humaine, l'insuline humaine, la chaîne A d'insuline humaine, la chaîne B d'insuline humaine, la pré-pro-insuline humaine, la pro-insuline humaine, l'hormone de croissance bovine, l'hormone de croissance porcine, l'interféron, le facteur libérant l'hormone de croissance, l'interleucine II, une enzyme ou une hormone.

8. Vecteur d'expression d'ADN recombinant selon la revendication 7, caractérisé en ce que la séquence de polypeptides fonctionnels code la pro-insuline humaine.

9. Plasmide du type illustré en figure 1, dénommé pOW440, pouvant être obtenu à partir de Bacillus subtilis MI112/pOW440 (NRRL B—15887).

10. Plasmide du type illustré en figure 2, dénommé pOW448, isolé de E. coli K12 JA221/pOW448, produit en transformant E. coli K12 JA221 avec les produits de digestion ligaturés d'EcoRI des plasmides pOW341 et pOW430 définis ci-après.

11. Plasmide pOW430, pouvant être obtenu à partir de Bacillus subtilis MI112/pOW430 (NRRL B—15833).

12. Plasmide pOW650 obtenu par ligature du fragment de XhoII à environ 0,65 kb du plasmide pOW440 défini dans la revendication 9 et du produit de digestion du plasmide pKC7 par BamHI et BglII (à partir de ATCC 37084).

13. Plasmide pOW050, obtenu par remplissage à la fois dans du site de BamHI du plasmide pBR322 et du fragment HpaII d'environ 1,4 kb contenant le gène de nucléase staphylococcique du plasmide pFOG301, en utilisant l'enzyme de Klenow, puis en ligaturant les fragments à extrémités émoussées résultants.

14. Plasmide pOW430 obtenu en ligaturant la séquence d'ADN appropriée, le fragment de HphI-XhoII à environ 0,27 kb du plasmide pNM587.4—4 (pouvant être obtenu à partir d'E. coli JA221/pNM587. 4—4, NRRL B—15812) et le produit de digestion de pUC8 par BamHI.

15. Plasmide du type illustré en figure 3, dénommé pOW341, pouvant être obtenu à partir d'E. coli K12 JA221/pOW341, produit en ligaturant le produit de mise à digestion du plasmide pOW650 défini dans la revendication 12 par BglII et le produit de digestion du plasmide pOW340 défini dans la revendication 14 par BamHI et ne transformant E. coli K12 JA221.

16. Cellule hôte transformée en vue de l'utiliser dans la méthode de la revendication 1 ou 2, comprenant un vecteur d'expression d'ADN selon l'une quelconque des revendications 5 à 15.

17. Cellule hôte transformée par un vecteur d'expression d'ADN selon l'une quelconque des revendications 5 à 15.

18. Cellule hôte selon la revendications 17, caractérisée en ce qu'elle est Bacillus ou E. coli.

19. Cellule hôte selon la revendications 17, caractérisée en ce qu'elle est Bacillus subtilis.

20. Cellule hôte d'E. coli transformée par un plasmide selon l'une quelconque des revendications 11 à 15.

21. Cellule hôte transformée choisie parmi le groupe comprenant: Bacillus subtilis MI112, transformée par le plasmide pOW440 défini dans la revendication 9, Bacillus subtilis MI112 transformé par la plasmide pOW430 défini dans la revendication 11, Bacillus subtilis SR22 transformé par le plasmide pOW448 défini dans la revendications 10, Bacillus subtilis MI112 transformé par le plasmide pOW448, Bacillus subtilis SR22 transformé par le plasmide pOW440, E. coli K12 JA221 transformé par le plasmide pOW448, E. coli K12 JA221 transformé par le plasmide pOW341 défini dans la revendication 15, E. coli K12 JA221 transformé par le plasmide pOW340 défini dans la revendication 14, E. coli K12 JA221 transformé par le plasmide pOW650 défini dans la revendication 12 ou Bacillus subtilis MI112 transformé par le plasmide pOW430.

# FIG.I

Restriction Site Map of
Plasmid pOW440
(5.7kb)

# FIG.2

Restriction Site Map of
Plasmid pOW448
(9.5kb)

# FIG.3

Restriction Site Map of
Plasmid pOW341
(5.3kb)